# EUROPEAN PATENT APPLICATION

(11) **EP 4 813 225 A1**
(43) Date of publication of application: **30.09.2026**
(21) Application number: 24893093.5
(22) Date of filing: 17.10.2024
(51) Int. Cl.: A24F 40/10, A24F 40/46, A24F 40/70

(54) **ATOMIZATION CORE AND PREPARATION METHOD THEREFOR, AND ATOMIZER**

(30) Priority: 24.11.2023 CN 202311589604
(71) Applicant: Smoore International Holdings Limited, Grand Cayman, KY1-1111 (KY)
(72) Inventor: HE, Xueqin, Grand Cayman KY1-1111, Cayman Islands (KY); CHEN, Wu, Grand Cayman KY1-1111, Cayman Islands (KY); MA, Xiaoguang, Grand Cayman KY1-1111, Cayman Islands (KY); LYU, Ming, Grand Cayman KY1-1111, Cayman Islands (KY); LIAO, Zhen, Grand Cayman KY1-1111, Cayman Islands (KY); ZHAO, Kai, Grand Cayman KY1-1111, Cayman Islands (KY); LIU, Yuying, Grand Cayman KY1-1111, Cayman Islands (KY); ZHANG, Lili, Grand Cayman KY1-1111, Cayman Islands (KY); XIAO, Congwen, Grand Cayman KY1-1111, Cayman Islands (KY); TANG, Genchu, Grand Cayman KY1-1111, Cayman Islands (KY)
(74) Representative: Winter, Brandl - Partnerschaft mbB
(86) International application number: PCT/CN2024/125460
(87) International publication number: WO 2025/107934

(57) **Abstract**

The present disclosure provides an atomizer core and a preparation method therefor, and an atomizer. The atomizer core includes a porous substrate having the atomizing surface and used for guiding an atomized substrate to the atomizing surface; and a heating element arranged on the atomizing surface of the porous substrate, wherein at least one surface of the heating element has a microstructure. The atomizer core accelerates a liquid supply speed, reduces risk of scaling of the atomizer core, and improves atomization efficiency and a puff taste.

## Description

### CROSS-REFERENCE TO RELATED APPLICATIONS

The present disclosure is based on and claims the priority to the Chinese Patent Application No. 202311589604X filed on November 24, 2023, which is incorporated herein in its entirety by reference.

### TECHNICAL FIELD

The present disclosure relates to the technical field of atomization, and in particular, to an atomizer core and a preparation method therefor, and an atomizer.

### BACKGROUND

An atomizer core usually includes a porous substrate and a heating element. The porous substrate is used for guiding an atomized substrate. The heating element is arranged on the porous substrate and is used for heating and atomizing the atomized substrate, to generate aerosols.

However, when the existing atomizer core is used, liquid supply is insufficient, the heating element is caused to generate the high temperature, and the atomizer core is likely to scale. As a result, the atomizing efficiency and a puff taste are affected.

### SUMMARY

The present disclosure provides an atomizer core and a preparation method therefor, and an atomizer for solving the problems that liquid supply of an atomizer core is insufficient, a heating element is caused to generate the high temperature, the atomizer core is likely to scale, and atomizing efficiency and a puff taste are affected.

To solve the technical problem, the present disclosure adopts a technical solution as follows: an atomizer core is provided. The atomizer core includes a porous substrate and a heating element, where the porous substrate has the atomizing surface and is used for guiding an atomized substrate to the atomizing surface; and the heating element is arranged on the atomizing surface of the porous substrate, and at least one surface of the heating element has a microstructure.

The heating element includes a heating substrate and an adsorption layer, and the adsorption layer at least covers the surface of the side, facing away from the porous substrate, of the heating substrate; and the surface of the heating substrate and/or the surface of the adsorption layer have/has the microstructure.

The surface roughness of the side, facing away from the heating substrate, of the adsorption layer is greater than the surface roughness of the heating substrate.

The adsorption layer includes a main body layer and a plurality of particles embedded in the main body layer.

The part of each particle protrudes from the surface of the side, facing away from the heating substrate, of the main body layer.

The D50 particle size of each particle is 7 um to 60 um; and/or the thickness of the main body layer is 5 um to 50 um.

The surface roughness of the heating element is 0.2 um to 10 um.

The melting point/the softening point of a constituent material of the particles is higher than the melting point/the softening point of a constituent material of the main body layer.

The adsorption layer covers the entire outer surface of the heating substrate in contact.

To solve the technical problem, the present disclosure adopts another technical solution as follows: an atomizer is provided. The atomizer includes the atomizer core described above.

To solve the technical problem, the present disclosure adopts yet another technical solution as follows: a preparation method for an atomizer core is provided. The method includes: providing a porous substrate; and forming a heating element on the porous substrate, where at least one surface of the heating element has a microstructure.

The arranging a heating element on the porous substrate includes: attaching the slurry to the entire outer surface of the heating substrate; and sintering the heating substrate to which the slurry is attached and the porous substrate together, to form the atomizer core; or arranging the heating substrate on the porous substrate; and attaching the slurry to the heating substrate and performing sintering, to form an adsorption layer on the surface of the heating substrate, where the adsorption layer at least covers the surface of the side, facing away from the porous substrate, of the heating substrate.

The slurry includes the following components in percentage by mass: 20% to 60% of glass powder, 15% to 40% of particles, 0.5% to 5% of dispersants, and the balance being organic carrier; the melting point/the softening point of the glass powder is lower than the melting point/the softening point of a constituent material of the particles; and the D50 particle size of the particles is greater than the D50 particle size of the glass powder.

Embodiments of the present disclosure have the beneficial effects as follows: different from the prior art, the atomizer core according to the embodiments of the present disclosure includes the porous substrate and the heating element. The porous substrate has the atomizing surface and is used for guiding the atomized substrate to the atomizing surface. The heating element is arranged on the atomizing surface of the porous heating element substrate, and the at least one surface of the heating element has the microstructure. The microstructure is arranged on the at least one surface of the heating element. Thus, capillary force of the surface of the heating element can be improved, the atomized substrate can be quickly supplied to the surface of the heating substrate, and risk of high temperature generation by the heating substrate due to insufficient liquid supply is reduced. Further, the surface area of the heating element can be increased, and the heating element can be in contact with more atomized substrates. Thus, the heat utilization rate is improved, risk of high temperature generation by the heating element is reduced, and then risk of scaling of the atomizer core is reduced. Further, when the atomizer core scales, a deposit is attached to the surface of the microstructure first, and a bonding strength of the deposit on the microstructure is poor due to the high surface roughness of the microstructure. Under the capillary force of the microstructure, the atomized substrate flows quickly, particular impact is generated on the deposit, and the deposit is caused to fall off the adsorption layer. Thus, risk of impact on the atomization efficiency and the puff taste due to deposit accumulation on the surface of the heating element is further reduced.

### BRIEF DESCRIPTION OF THE DRAWINGS

FIG. 1 is a schematic structural diagram of an atomizer according to an embodiment of the present disclosure;
FIG. 2 is a schematic diagram of overall scaling of an atomizer core according to an embodiment of the present disclosure;
FIG. 3 is a schematic diagram of an exploded view of the atomizer core shown in FIG. 2 according to an embodiment of the present disclosure;
FIG. 4 is a schematic diagram of a vertical section of an atomizer core according to an embodiment of the present disclosure;
FIG. 5 is a schematic diagram of a vertical section of an atomizer core according to another embodiment of the present disclosure;
FIG. 6 is a schematic structural diagram of an adsorption layer according to an embodiment of the present disclosure; and
FIG. 7 is a flowchart of a preparation method for an atomizer core according to an embodiment of the present disclosure.

1-atomizer core; 11-porous substrate; 12-heating element; 120-microstructure; 121-heating substrate; 121a-heating portion; 121b-first connection portion; 121c-second connection portion; 122-adsorption layer; 123-main body layer; 124-particle; 2-airflow channel; and 3-liquid storage bin.

### DETAILED DESCRIPTION

Technical solutions in embodiments of the present disclosure will be described clearly and completely below in conjunction with accompanying drawings in the embodiments of the present disclosure. Apparently, the embodiments described are merely some embodiments rather than all embodiments of the present disclosure. All other embodiments derived by those of ordinary skill in the art from the embodiments of the present disclosure without creative efforts should fall within the protection scope of the present disclosure.

In the present disclosure, the terms "first", "second", and "third" are merely used for describing purposes and may not be understood as indicating or implying relative importance or implicitly indicating the number of indicated technical features. Thus, the features defined with "first", "second", and "third" can explicitly or implicitly include at least one of the features. In the description of the present disclosure, "plurality" indicates at least two, for example, two and three unless otherwise explicitly and specifically defined. All directional indications (for example, up, down, left, right, front, and rear...) in the embodiments of the present disclosure are merely used for explaining relative positional relationships, motion conditions, etc. between components in a particular specific posture (as shown in the accompanying drawings), and if the specific posture changes, the directional indications also change accordingly. In addition, the terms "comprise", "include", "has", and "have" and their any form of variation are intended to cover non-exclusive inclusions. For example, a process, a method, a system, a product, or a device that includes a series of steps or units is not limited to listed steps or modules, but may further include optionally a step or a module that is not listed, or may further include optionally other steps or units inherent to the process, the method, the product, or the device.

Reference to the "embodiment" herein means that a specific feature, structure, or characteristic described in conjunction with the embodiment may be included in at least one embodiment of the present disclosure. The appearance of this phrase in different places throughout the description does not necessarily mean the same embodiment, or an independent or alternative embodiment mutually exclusive with other embodiments. Those of ordinary skill in the art understood explicitly or implicitly that the embodiments described herein can be combined with other embodiments.

In the related art, components of the atomized substrate usually include propylene glycol, glycerol, essence, nicotine salt, tobacco extracts, and other additives. These components are likely to boil and atomize at high temperature, to generate aerosols. Through long-term research, the inventor of the present disclosure finds that boiling points and fluidity of different atomized substrates are different due to different compositions and ratios. When atomized with the same atomizer core, some atomized substrates may be consumed too quickly or in short supply during an atomization process due to low boiling points or poor fluidity. As a result, the atomizer core rises in temperature, and lots of deposits (such as soot) are generated. In addition, macromolecular substances such as the tobacco extracts and the additives are likely to generate the deposit when decomposed in the atomization process, and the deposit is accumulated on the surface of heating element with the following problems.

Firstly, the deposit may block contact between a heating element and the atomized substrate, and main components of the deposit are carbon (C) and oxygen (O). Thus, heat generated by the heating element cannot be conducted to the atomized substrate in time, and the atomization efficiency is affected.

Secondly, the temperature of the heating element increases gradually, and generation of the deposit is further increased. Finally, along with puffs, the deposit on the surface of the heating element gradually increases, the atomization efficiency gradually decreases, puff experience gradually deteriorates, and even a burned taste occurs.

Thirdly, after accumulated on the surface of the heating element, the deposit may be heated repeatedly during a puff process, and the abnormal high temperature may be generated. Thus, the deposit or E-liquid is caused to decompose abnormally, to generate harmful aldehydes and ketones.

Based on this, the embodiments of the present disclosure provide an atomizer core. Through the atomizer core, a liquid supply rate can be increased, and risk of high temperature generation of a heating substrate caused by insufficient liquid supply can be reduced. In addition, generation of the deposit on the surface of the atomizer core during continuous heating is reduced, and generation of the harmful aldehydes and ketones is reduced.

The present disclosure will be described in detail below with reference to accompanying drawings and in conjunction with embodiments.

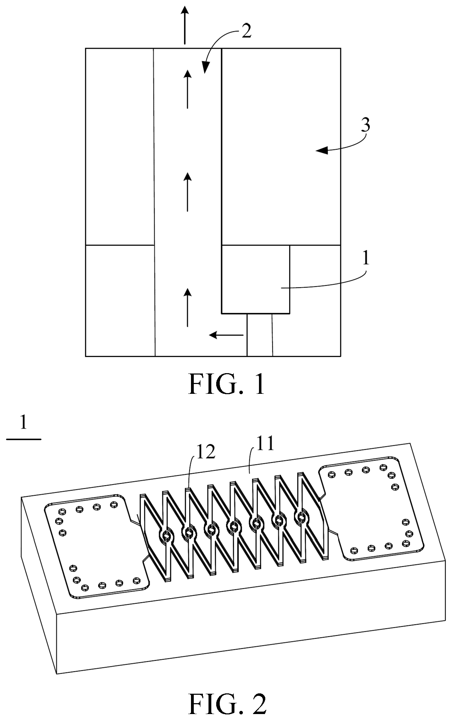

In this embodiment, with reference to FIG. 1, FIG. 1 is a schematic structural diagram of an atomizer according to an embodiment of the present disclosure. The atomizer is provided. The atomizer may be used specifically in different fields, such as medical treatment, beauty, and recreational puffs. In a specific embodiment, the atomizer may be used in an electronic atomizing device, and is used for atomizing an atomized substrate and generating aerosols for a puffer to puff. The following embodiments take the recreational puff as an example.

The atomizer includes an atomizer core 1, an airflow channel 2, a suction nozzle, and a liquid storage bin 3. The airflow channel 2 communicates with the suction nozzle and the atomizer core 1. The liquid storage bin 3 is used for storing the atomized substrate. The atomizer core 1 communicates with the liquid storage bin 3, and is used for atomizing the atomized substrate, to generate aerosols. The aerosol generated through atomization flows to the suction nozzle through the airflow channel 2 for a user to puff. For a specific structure and function of the atomizer core 1, reference can be made to a specific structure and function of the atomizer core 1 in the following embodiments, and the same or similar technical effects can be achieved, which is not repeated herein.

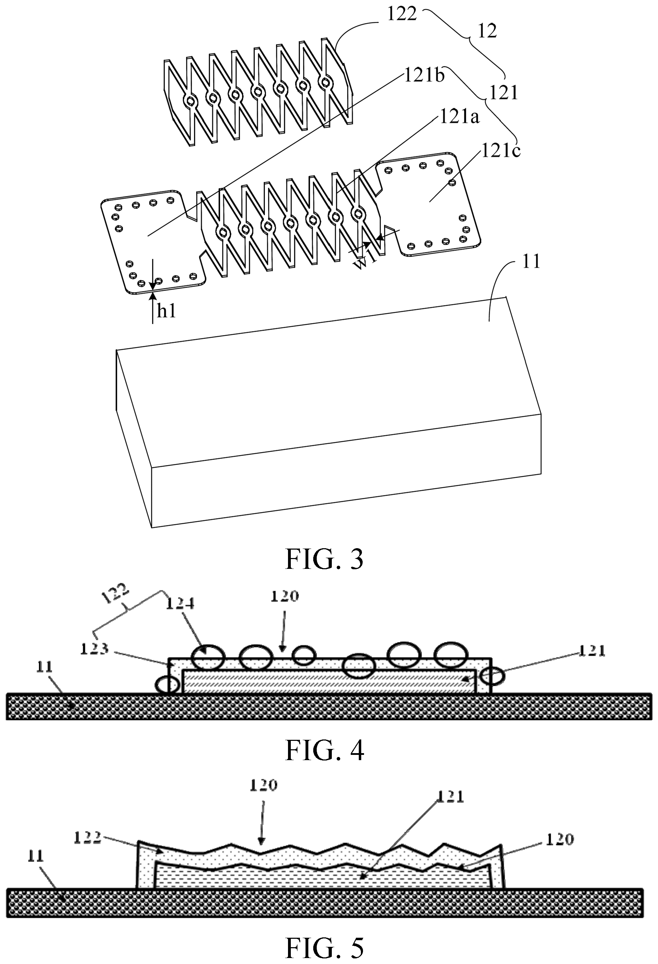

With reference to FIG. 2 to FIG. 4, FIG. 2 is a schematic diagram of overall scaling of an atomizer core according to an embodiment of the present disclosure. FIG. 3 is a schematic diagram of an exploded view of the atomizer core shown in FIG. 2 according to an embodiment of the present disclosure.

FIG. 4 is a schematic diagram of a vertical section of an atomizer core according to an embodiment of the present disclosure. In this embodiment, an atomizer core 1 is provided. The atomizer core 1 includes a porous substrate 11 and a heating element 12.

The porous substrate 11 has a plurality of micropore structures, and each micropore structure has capillary force. Specifically, porosity of the porous substrate 11 is greater than or equal to 35% and is less than and equal to 80%. For example, the porosity may be 35%, 40%, 45%, 50%, 60%, 70%, or 80%. The porous substrate 11 having such a porosity range has particular liquid conductivity, so as to improve an effect of liquid supply to the heating element 12. In addition, a pore structure of the porous substrate 11 can absorb particular deposits (such as soot) in the pore structure, thus reducing impact of the deposit on the heating element 12.

An average pore diameter of the porous substrate 11 is greater than or equal to 3 um and is less than or equal to 50 um. For example, the average pore diameter of the porous substrate 11 may be 3 um, 10 um, 15 um, 20 um, 30 um, 40 um, or 50 um.

The porous substrate 11 has a liquid absorption surface and an atomizing surface, and the liquid absorption surface may have a liquid discharging tank. The liquid discharging tank communicates with the liquid storage bin 3, the atomized substrate in the liquid storage bin 3 is guided to the liquid discharging tank, and the atomized substrate in the liquid discharging tank is guided to the atomizing surface of the porous substrate 11 based on the capillary force of the porous substrate 11.

Materials of the porous substrate 11 may include one or more of alumina, silicon oxide, silicon nitride, silicate, hydroxyapatite, and silicon carbide. For example, the porous substrate 11 may be a ceramic porous substrate, a glass porous substrate, a polymer porous substrate, etc. In this embodiment, the porous substrate 11 is porous ceramic, and disordered pores formed in a preparation process of the porous ceramic have capillary force. In other implementations, the porous substrate 11 has other porous structures. For example, a plurality of through holes are provided in a dense substrate, to form a porous structure, and the dense substrate may be dense ceramics, glass, etc. Specifically, the porous substrate 11 may be a regular cuboid or cube, or other irregular three-dimensional structures, which is not limited in the present disclosure.

The heating element 12 is arranged on the atomizing surface of the porous substrate 11, and is used for heating and atomizing the atomized substrate when energized, to form the aerosols. At least one surface of the heating element 12 has a microstructure 120. The microstructure 120 means that the surface of the material is uneven and granular. The microstructure 120 is a low surface energy structure similar to a hydrophobic effect of a lotus leaf, and has particular capillary force.

With the arrangement, by forming the granular undulating microstructure 120 on the surface of the heating element 12, due to the capillary force, the undulating microstructure 120 can easily store and quickly absorb the atomized substrate. Thus, the heating element 12 is prevented from generating the high temperature due to insufficient liquid supply. In addition, the microscopic area of the surface of the heating element 12 can be increased, and the surface of the heating element 12 can be in contact with more atomized substrates. Thus, the energy utilization rate can be improved, the probability of generating the high temperature can be reduced, and finally generation of the deposit can be reduced. In addition, the coarse microstructure 120 has uniform protruding particles, and a gap exits between particles. When the deposit is generated, the deposit is attached to the surfaces of particles first. Due to the discontinuous phenomenon between the particles, the contact area between the deposit and microstructure 120 is small, and the bonding strength is poor. Under the capillary force between the particles, the atomized substrate flows rapidly, and has particular impact on the deposit, and the deposit falls off. Thus, deposit accumulation on the surface of the heating element 12 is further reduced, and risk of impact on atomization efficiency and a puff taste due to deposit accumulation on the surface of the heating element 12 is further reduced.

In an embodiment, as shown in FIG. 3, the heating element 12 includes a heating substrate 121 and an adsorption layer 122.

The heating substrate 121 is arranged on the atomizing surface of the porous substrate 11, and is used for heating to atomize the atomized substrate when energized, to form the aerosols. The heating substrate 121 includes a heating portion 121a, a first connection portion 121b, and a second connection portion 121c. The first connection portion 121b and the second connection portion 121c are connected to the two sides of the heating portion 121a respectively. The first connection portion 121b is used for being in contact with and electrically connected to a positive electrode, and the second connection portion 121c is used for being in contact with and electrically connected to a negative electrode. A main body of the electronic atomizing device electrifies the heating portion 121a through the positive electrode and the negative electrode, and the heating portion 121a is energized, to generate heat, to atomize the atomized substrate.

The heating portion 121a may have a filament structure, a strip structure, or a grid structure. Specifically, the heating portion 121a may be distributed in a grid shape, to increase an contact area between the heating portion 121a and the porous substrate 11 within the effective atomizing surface area. Materials of the heating substrate 121 include at least one of metal alloy sheets such as an iron-chromium alloy, an iron-chromium-aluminum alloy, an iron-chromium-nickel alloy, an chromium-nickel alloy, a titanium alloy, a stainless steel alloy, a Karma alloy, and a precious metal alloy.

In a specific embodiment, the thickness h1 of the heating substrate 121 is greater than or equal to 0.01 mm and is less than or equal to 2.00 mm. For example, the thickness h1 of the heating substrate 121 is 0.01 mm, 0.1 mm, 0.5 mm, 1.0 mm, 1.5 mm, 2.0 mm, etc. The width w1 of the heating portion 121a of the heating substrate 121 is greater than or equal to 0.05 mm and is less than or equal to 3 mm. For example, the width w1 of the heating portion 121a is 0.05 mm, 0.1 mm, 0.5 mm, 1.0 mm, 1.5 mm, 2.0 mm, 2.5 mm, 3.0 mm, etc.

The adsorption layer 122 at least covers the surface of the side, facing away from the porous substrate 11, of the heating substrate 121. The surface of the heating substrate 121 and/or the surface of the adsorption layer 122 have/has the microstructure 120. In some embodiments, with reference to FIG. 4, each of the surfaces of the sides, facing away from the porous substrate 11, of the heating substrate 121 and the heating substrate 121 has the microstructure 120. In this way, the capillary force on the surface of the heating element 12 can be further improved, the liquid supply rate can be further increased, and insufficient liquid supply can be prevented.

In an embodiment, with reference to FIG. 4, the surface roughness of the surface of the side, facing away from the heating substrate 121, of the adsorption layer 122 is greater than the surface roughness of the surface of the side, in contact with the adsorption layer 122, of the heating substrate 121. In this way, the surface area of the heating element 12 can be increased, the binding force of the deposit on the heating element 12 can be reduced, and the risk of the impact on the atomization efficiency and the puff taste due to deposit accumulation on the surface of the heating element 12 can be further reduced. The heating substrate 121 is usually made of metal sheets with the low surface roughness, and the overall surface roughness of the heating element 12 is improved through the arrangement of the adsorption layer 122.

With reference to FIG 5, FIG. 5 is a schematic diagram of a vertical section of an atomizer core according to another embodiment of the present disclosure. The surface of a heating substrate 121 is treated to form a particular surface microstructure 120, that is, the heating substrate 121 has the high roughness. The surface of the side, facing away from the heating substrate 121, of an adsorption layer 122 also has a microstructure 120. In this embodiment, the microstructure 120 on the surface of the adsorption layer 122 may be formed merely based on the microstructure 120 of the original heating substrate 121. That is, the adsorption layer 122 mainly includes a main body layer 123. That is, the adsorption layer 122 does not have the microstructure protruding from the surface, but mainly forms the surface roughness of a heating element 12 through the microstructure 120 of the heating substrate 121. In another embodiment, the adsorption layer 122 further includes a plurality of particles 124 embedded in the main body layer 123. The microstructure of the surface of the heating substrate 121 may be prepared by any method such as laser etching, chemical etching, and rolling. Specifically, the surface roughness of the heating element 12 is 0.2 um to 10 um. That is, the surface roughness of the adsorption layer 122 is 0.2 um to 10 um. For example, the surface roughness of the heating element 12 is 0.2 um, 1.0 um, 3.0 um, 5.0 um, 8.0 um, or 10 um. The surface roughness refers to the roughness of the processed surface with small spacing and tiny peaks and valleys. The distance (wave pitch) between two peaks or two valleys is very small (1 mm or below), as a micro-geometric error. The smaller the surface roughness is, the smoother the surface is.

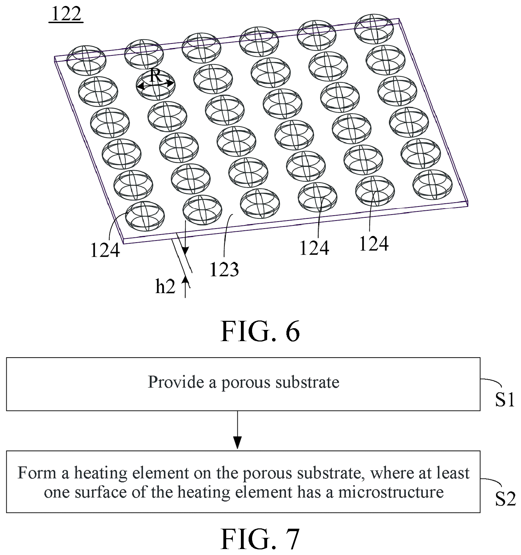

In an embodiment, with reference to FIG. 6 and FIG. 4, FIG. 6 is a schematic structural diagram of an adsorption layer 122 according to an embodiment of the present disclosure. The adsorption layer 122 includes a main body layer 123 and a plurality of particles 124 embedded in the main body layer 123.

In a specific embodiment, the main body layer 123 includes a glass glaze layer, and components of the glass glaze layer do not include heavy metal elements such as lead (Pb), arsenic (As), chromium (Cr), and cadmium (Cd). Thus, harm to the health of the user caused since heavy metal ions are generated during a heating process, and are puffed by the user is avoided.

The glass glaze layer is a dense glass layer generated by sintering raw glass powder particles at a particular temperature. The sintering temperature is usually higher than the softening point of raw glass powder particles, and the thickness of the glass glaze layer is mainly affected by the thickness of a coating and the D50 particle size of the raw glass powder.

The particle 124 may include one compound or a mixture of several compounds such as alumina, zirconia, silicon carbide, and silicon oxide. The D50 particle size of the particle 124 is 7 um to 60 um. For example, the D50 particle size of the particle 124 is 7 um, 15 um, 25 um, 35 um, 45 um, or 60 um.

In a specific embodiment, as shown in FIG. 4, the part of the particle 124 is embedded in the main body layer 123, and the remaining part protrudes from the surface of the side, facing away from the heating substrate 121, of the main body layer 123. In this way, the rough surface of the heating element 12 can be formed, and the heating element 12 presents morphology of an undulating microstructure 120. Thus, based on the idea, to reliably realize the microstructure 120, the D50 particle size of the particle 124 should be greater than the thickness of the main body layer 123, to ensure that the at least parts of most of the particles 124 at least partially protrude from the main body layer 123 and form the uniform microstructure 120. In addition, to ensure that the shape of the particle 124 is controllable in a preparation process, the melting point/the softening point of a constituent material of the particle 124 is higher than the melting point/the softening point of a constituent material of the main body layer 123. That is, the melting point/softening point of the raw material particles of the particles 124 is higher than the melting point/the softening point of the raw material glass powder of the main body layer 123. In a process that the raw glass powder of the main body layer 123 is softened to form the dense glaze layer, the raw particles of the particle 124 may still maintain the original particle size, such that the part of the particle 124 may protrude from the surface of the main body layer 123. The particle 124 may be composed of a single material or a mixture of a plurality of materials.

Specifically, the thickness h2 of the main body layer 123 is 5 um to 50 um. For example, the thickness h2 of the body layer 123 is 5 um, 10 um, 20 um, 30 um, 40 um, or 50 um. In a specific embodiment, the adsorption layer 122 covers the plurality of outer surfaces of the heating substrate 121. Most preferably, the adsorption layer 122 covers the entire outer surface of the heating substrate 121. The heating substrate 121 is in contact with the porous substrate 11 through the adsorption layer 122. For the heating element 12 in this embodiment, coating slurry forming the microstructure 120 may be attached to the entire outer surface of the heating substrate 121 through spraying, screen printing, soaking, etc. Then the heating element 12 is combined with the porous substrate 11 through a process of slip casting or powder pressing molding, and sintering is performed to obtain the atomizer core 1.

In other embodiments, the adsorption layer 122 may alternatively cover all the other surfaces of the heating substrate 121 except the bonding surface certainly. The bonding surface of the heating substrate 121 refers to the surface of the side, oriented towards the porous substrate 11, of the heating substrate 121. In this embodiment, the heating substrate 121 may be molded and sintered with the porous substrate 11. Then the coating slurry provided with the microstructure 120 may be attached to the surface of the heating substrate 121 through spraying or screen printing. Finally, the atomizer core 1 may be obtained through sintering.

It should be noted that in some embodiments, the porous substrate 11 and the heating element 12 may be fixed through direct embedding or be bonded through an adhesive layer. The adhesive layer may be a glass layer, etc.

The coating slurry for forming the microstructure 120 includes the following components in percentage by mass: 20% to 60% of glass powder, 15% to 40% of high-melting-point particles 124, 0.5% to 5% of dispersants, and the balance being organic carrier. The glass powder may be low-temperature glass such as SnO-ZnO-P₂O₅ ternary glass (SZP ternary glass system) and an alkali borosilicate glass system. The particles 124 may be spherical alumina particles. The organic carrier may include an organic solvent and thermoplastic resin. The organic carrier may make the glass powder and the particle 124 have appropriate fluidity and plasticity. The organic solvent may be at least one of butyl carbitol, terpineol, and butyl carbitol acetate. The dispersant may adjust the stability of the glass powder, and the dispersant may adopt polyethylene wax, paraffin wax, or other commercially available dispersants. The commercially available dispersant may be BYK-110, etc. BYK-110 is one of commercial dispersants, and is mainly a copolymer solution having acidic groups, and the solvents are propylene glycol methyl ether acetate and alkyl benzene. It should be noted that the atomizer core 1 finally obtained has no organic carriers or dispersants or has a small amount of organic carriers and dispersants.

The atomizer core 1 according to this embodiment includes the porous substrate 11 and the heating element 12. The porous substrate 11 has the atomizing surface and is used for guiding an atomized substrate to the atomizing surface. The heating element 12 is arranged on the atomizing surface of the porous substrate 11, and at least one surface of the heating element 12 has a microstructure 120. The microstructure 120 is arranged on the at least one surface of the heating element 12. Thus, capillary force of the surface of the heating element 12 can be improved, the atomized substrate can be quickly supplied to the surface of the heating substrate 121, and risk of high temperature generation by the heating substrate 121 due to insufficient liquid supply is reduced. Further, the surface area of the heating element 12 can be increased, and the heating element 12 can be in contact with more atomized substrates. Thus, the heat utilization rate is improved, risk of high temperature generation by the heating element 12 is reduced, and then risk of scaling of the atomizer core 1 is reduced. Further, when the atomizer core 1 scales, a deposit is attached to the surface of the microstructure 120 first, and a bonding strength of the deposit on the microstructure 120 is poor due to the high surface roughness of the microstructure 120. Under the capillary force of the microstructure 120, the atomized substrate flows quickly, particular impact is generated on the deposit, and the deposit is caused to fall off the microstructure 120. Thus, risk of impact on the atomization efficiency and the puff taste due to deposit accumulation on the surface of the heating element 12 is further reduced. In addition, the risk that the deposit is heated repeatedly, and the abnormal high temperature is generated, causing the deposit or E-liquid to decompose abnormally, to generate harmful aldehydes and ketones is reduced.

With reference to FIG. 7, FIG. 7 is a flowchart of a preparation method for an atomizer core according to an embodiment of the present disclosure. In this embodiment, a preparation method for an atomizer core is provided. The preparation method may be used for preparing the atomizer core 1 according to the embodiment, and the preparation method specifically includes:
Step S1: A porous substrate is provided.

The porous substrate 11 has a plurality of micropore structures, and each micropore structure has capillary force, and is used for guiding a liquid. Reference can be made to the description above for the specific structure and function of the porous substrate 11. With reference to FIG. 3, the heating substrate 121 is used for heating and atomizing an atomized substrate when energized. Reference can be made to the description above for the specific structure and function of the heating substrate 121.

Step S2: A heating element is formed on the porous substrate. At least one surface of the heating element has a microstructure.

In a specific embodiment, step S2 specifically includes: step S21: a heating substrate 121 and slurry are provided.

The slurry includes 20% to 60% of glass powder, 15% to 40% of high-melting-point particles 124, 0.5% to 5% of dispersants, and the balance being organic carrier. The D50 particle size of the particle 124 is greater than the D50 particle size of the glass powder. In this way, the microstructure 120 of the surface of the heating element 12 may be generated by using the particle 124, and the surface roughness of the adsorption layer 122 generated and including the particles 124 may be within a preset range.

The softening temperature of the glass powder is 400°C to 1200°C, such as 400°C, 500°C, or 600°C. The D50 particle size of the glass powder is 2 um to 30 um, such as 2 um, 5 um, 10 um, 20 um, or 30 um. The specific size may be selected according to actual needs. The glass powder may be low-temperature glass such as SnO-ZnO-P₂O₅ ternary glass (SZP ternary glass system) and an alkali borosilicate glass system. The particles 124 may be spherical alumina particles. The organic carrier may include an organic solvent and thermoplastic resin. The organic carrier may make the glass powder and the particle 124 have appropriate fluidity and plasticity. The organic solvent may be at least one of butyl carbitol, terpineol, and butyl carbitol acetate. The dispersant may adjust the stability of the glass powder, and the dispersant may adopt BYK-110, polyethylene wax, paraffin wax, etc. It should be noted that the atomizer core 1 finally obtained has no organic carriers or dispersants or has a small amount of organic carriers and dispersants.

The melting point/the softening point of the glass powder is lower than the melting point/the softening point of a constituent material of the particles 124. In this way, it can be guaranteed that in a case of the particular temperature, for example, in the sintering process, the glass powder is melted to form the main body layer 123. The particles 124 are not melted or melted to the low melting degree, to form the adsorption layer 122 presenting morphology of the undulating microstructure 120 and having the high surface roughness on the surface of the heating substrate 121 (shown in FIG. 6). That is, the sintering temperature should be higher than the softening point stability of the glass powder and be lower than the melting point/softening point temperature of the constituent material of the particles 124.

Step S22: The slurry is attached to the entire outer surface of the heating substrate 121.

Specifically, the slurry may be attached to the entire outer surface of the heating substrate 121 through spraying, screen printing, soaking, etc.

Step S23: The heating substrate 121 to which the slurry is attached and the porous substrate 11 are sintered together, to form the atomizer core 1.

Specifically, the heating substrate 121 to which the slurry may be attached and the porous substrate 11 are sintered together through a process of slip casting or powder pressing molding. Then the heating substrate 121 and the porous substrate 11 are sintered, to obtain the atomizer core 1.

In another specific embodiment, step S2 specifically includes: step S21': a heating substrate 121 and slurry are provided.

The slurry is the slurry provided in step S21. Step S21 may alternatively be performed before step S1.

Step S22': The heating substrate 121 is arranged on the porous substrate 11.

The heating substrate 121 and the porous substrate 11 are molded and sintered together through a process of slip casting or powder pressing molding.

Step S23': The slurry is attached to the heating substrate 121, and sintering is performed to form an adsorption layer 122 on the surface of the heating substrate 121.

Specifically, the slurry may be attached to all the other surfaces of the heating substrate 121 except the bonding surface through spraying or screen printing. Then the heating substrate 121 and the porous substrate 11 are sintered, to obtain the atomizer core 1. After the organic carrier and the dispersant are sintered, all or most of the organic carrier and the dispersant are volatilized. That is, the finally generated atomizer core 1 has no organic carriers or dispersants or has almost no organic carriers or dispersants.

Two specific experiments conducted by the inventor of the present disclosure to form the adsorption layer 122 on the surface of the heating substrate 121 are described below.

Experiment 1: a softening point of glass powder was 400°C to 600°C, the D50 particle size was 4 um, and the proportion of the glass powder in slurry was 38%. Particles 124 were spherical alumina particles, the D50 particle size was 10 um, and the proportion of the particles 124 in the slurry was 15%. The proportion of organic carrier in the slurry was 45%. A dispersant was BYK-110, and the proportion of the dispersant in the slurry was 2%. The proportion of thermoplastic resin in the organic carrier was 5%, and the proportion of an organic solvent was 95%. A preparation method for the organic carrier includes: heating was performed in a water bath at 60°C to 80°C, the thermoplastic resin was completely dissolved into the organic solvent, and then filtering was performed through filter cloth of 200 meshes to 400 meshes, to finally obtain the organic carrier. The solvent may be butyl carbitol, terpineol, and butyl carbitol acetate. The glass powder, the particles 124 having the high melting point, and the dispersant were added into the organic carrier, and the slurry was obtained after uniform stirring. The slurry was sprayed to the surface of the heating substrate 121 and sintered at 750°C, to obtain the heating element 12. The surface of the heating element 12 had a microstructure 120 having the roughness of 0.5 um to 1.5 um. The D50 particle size represented the corresponding particle size when the cumulative particle size distribution percentage of a sample reached 50%. A physical meaning was that 50% of the particles were greater than the D50 particle size and 50% of the particles were less than the D50 particle size.

Experiment 2: a softening point of glass powder was 800°C to 1000°C, the D50 particle size was 10 um, and the proportion of the glass powder in slurry was 55%. High-melting-point particles 124 were spherical zirconia particles, the D50 particle size was 18 um, and the proportion of the particles 124 in the slurry was 30%. The proportion of organic carrier in the slurry was 14%. A dispersant was BYK-110, and the proportion of the dispersant in the slurry was 1%. The proportion of thermoplastic resin in the organic carrier was 30%, and the proportion of an organic solvent was 70%. A preparation method for the organic carrier includes: heating was performed in a water bath at 60°C to 80°C, the thermoplastic resin was completely dissolved into the organic solvent, and then filtering was performed through filter cloth of 200 meshes to 400 meshes, to finally obtain the organic carrier. The glass powder, the particles 124 having the high melting point, and the dispersant were added into the organic carrier, and the slurry was obtained after uniform stirring. The slurry was screen-printed to the surface of the heating substrate 121 and sintered at 1150°C, to form the heating element 12. The surface of the heating element 12 had a microstructure 120 having the roughness of 2 um to 5 um.

According to the preparation method for an atomizer core 1 provided by this embodiment, the porous substrate 11 is provided. Then, the heating element 12 is formed on the porous substrate 11. The at least one surface of the heating substrate 121 has the microstructure 120. According to the atomizer core 1 prepared by the method, capillary force of the surface of the heating element 12 can be improved, the atomized substrate can be quickly supplied to the surface of the heating substrate 121, and risk of high temperature generation by the heating substrate 121 due to insufficient liquid supply is reduced. Further, the surface area of the heating element 12 can be increased, and the heating element 12 can be in contact with more atomized substrates. Thus, the heat utilization rate is improved, risk of high temperature generation by the heating element 12 is reduced, and then risk of scaling of the atomizer core 1 is reduced. Further, when the atomizer core 1 scales, the deposit is attached to the surface of the microstructure 120 first, and the bonding strength of the deposit on the microstructure 120 is poor due to the high surface roughness of the microstructure 120. Under the capillary force of the microstructure 120, the atomized substrate flows quickly, particular impact is generated on the deposit, and the deposit is caused to fall off the microstructure 120. Thus, risk of impact on the atomization efficiency and the puff taste due to deposit accumulation on the surface of the heating element 12 is further reduced.

The descriptions above are merely preferred implementations of the present disclosure, and are not intended to limit the patent scope of the present disclosure. Any equivalent structure or equivalent process transformation made using the contents of the description and accompanying drawings of the present disclosure, or any direct or indirect application thereof in other related technical fields should equally fall within the patent protection scope of the present disclosure.

## Claims

1. An atomizer core, comprising:
a porous substrate having the atomizing surface and used for guiding an atomized substrate to the atomizing surface; and
a heating element arranged on the atomizing surface of the porous substrate, wherein at least one surface of the heating element has a microstructure.

2. The atomizer core of claim 1, wherein
the heating element comprises a heating substrate and an adsorption layer, and the adsorption layer at least covers the surface of the side, facing away from the porous substrate, of the heating substrate; and the surface of the heating substrate and/or the surface of the adsorption layer have/has the microstructure.

3. The atomizer core of claim 2, wherein
the surface roughness of the side, facing away from the heating substrate, of the adsorption layer is greater than the surface roughness of the heating substrate.

4. The atomizer core of claim 3, wherein
the adsorption layer comprises a main body layer and a plurality of particles embedded in the main body layer.

5. The atomizer core of claim 4, wherein
the part of each particle protrudes from the surface of the side, facing away from the heating substrate, of the main body layer.

6. The atomizer core of claim 4, wherein
the D50 particle size of each particle is 7 um to 60 um; and/or
the thickness of the main body layer is 5 um to 50 um.

7. The atomizer core of claim 2, wherein
the surface roughness of the heating element is 0.2 um to 10 um.

8. The atomizer core of claim 4, wherein
the melting point/the softening point of a constituent material of the particles is higher than the melting point/the softening point of a constituent material of the main body layer.

9. The atomizer core of claim 2, wherein
the adsorption layer covers the entire outer surface of the heating substrate.

10. An atomizer, comprising the atomizer core of any one of claims 1 to 9.

11. A preparation method for an atomizer core, comprising:
providing a porous substrate; and
forming a heating element on the porous substrate, wherein at least one surface of the heating element has a microstructure.

12. The preparation method for the atomizer core of claim 11, wherein
the forming a heating element on the porous substrate comprises:
providing a heating substrate and slurry; and
attaching the slurry to the entire outer surface of the heating substrate; and sintering the heating substrate to which the slurry is attached and the porous substrate together, to form the atomizer core; or arranging the heating substrate on the porous substrate; and attaching the slurry to the heating substrate and performing sintering, to form an adsorption layer on the surface of the heating substrate, wherein the adsorption layer at least covers the surface of the side, facing away from the porous substrate, of the heating substrate.

13. The preparation method for the atomizer core of claim 12, wherein
the slurry comprises the following components in percentage by mass: 20% to 60% of glass powder, 15% to 40% of particles, 0.5% to 5% of dispersants, and the balance being organic carrier; the melting point/the softening point of the glass powder is lower than the melting point/the softening point of a constituent material of the particles; and the D50 particle size of the particles is greater than the D50 particle size of the glass powder.
